# EUROPÄISCHE PATENTSCHRIFT

(11) **EP 2 855 651 B1**
(45) Veröffentlichungstag und Bekanntmachung des Hinweises auf die Patenterteilung: **02.11.2016**
(21) Anmeldenummer: 13727098.9
(22) Anmeldetag: 29.05.2013
(51) Int. Cl.: C11D 1/94, A61Q 5/02, A61K 8/42, A61Q 19/10, A61K 8/34, C11D 3/20, C11D 3/43

(54) **N-METHYL-N-ACYLGLUCAMIN ENTHALTENDE ZUSAMMENSETZUNG**
N-METHYL-N-ACYLGLUCAMINE-CONTAINING COMPOSITION
COMPOSITION CONTENANT DE LA N-MÉTHYL-N-ACYLGLUCAMINE

(30) Priorität: 30.05.2012 DE 102012010655
(43) Veröffentlichungstag der Anmeldung: 08.04.2015
(73) Patentinhaber: Clariant International Ltd, 4132 Muttenz (CH)
(72) Erfinder: KLUG, Peter, 63762 Großostheim (DE); MILDNER, Carina, 65931 Frankfurt am Main (DE)
(74) Vertreter: Holmes, Rosalind
(86) Internationale Anmeldenummer: PCT/EP2013/061065
(87) Internationale Veröffentlichungsnummer: WO 2013/178679

(56) Entgegenhaltungen:
- EP-A1- 1 078 978
- WO-A1-92/06161
- WO-A1-96/03974
- DE-C1- 4 435 383
- US-A- 2 703 798

## Beschreibung

Die Erfindung betrifft eine Zusammensetzung, enthaltend mindestens ein anionisches Tensid, ein Betaintensid, eine Mischung aus N-Methyl-N-acylglucaminen, ein Glycerin-Derivat, ein Lösungsmittel und gegebenenfalls ein oder mehrere Additive, sowie ein Verfahren zur Herstellung der Zusammensetzung. Ferner betrifft die Erfindung die Verwendung der Zusammensetzung zur Behandlung oder Pflege der Haut oder Haare z.B. als Shampoo, Gesichtsreiniger, Flüssigreiniger oder Duschbad.

Bei der Herstellung von Zusammensetzungen, insbesondere der Herstellung von Reinigungszusammensetzungen, sollten eine Reihe von Kriterien beachtet werden, wie beispielsweise eine gute reinigende Wirkung, ausreichende schäumende Eigenschaften, gute Hautverträglichkeit, ein gutes Gefühl in Bezug auf Haut, Haare und speziell keine Reizung der Schleimhäute. Haut und Haare bestehen aus mehren Schichten, die unter anderem Keratin und Kollagen als Faserproteine umfassen. Anionische Tenside können in die Schichten eindringen und diese zerstören. Ideale Reinigungsmittel für kosmetische oder pharmazeutische Anwendungen sollten die Haut oder das Haar sanft reinigen, ohne Entfettung und/oder Trocknen der Haare und der Haut und ohne zu reizen. Die meisten schäumenden Seifen, Dusch- und Badezusätze, Shampoos und Kosmetika versagen in dieser Hinsicht oder zeigen entsprechend schlechtes Schaumverhalten.

EP 0 550 637 A1 beschreibt ein Verfahren zur Herstellung von Polyhydroxyfettsäureamid-Materialien, welche unter anderem als Tenside verwendet werden können. Das Verfahren ist besonders dann nützlich, wenn das N-Alkylpolyhydroxyamin die Formel N(R¹)CH₂(CH₂OH)₄CH₂OH besitzt. Bei dem Verfahren wird bevorzugt ein C₁₂-C₂₀-Fettsäuremethylester verwendet. Ein bevorzugtes Verfahren zur Herstellung von Waschmitteltensiden ist eines, bei dem das N-Alkylpolyhydroxyamin ein N-Methylglucoamin ist, der Fettsäureester ein C₁₂-C₂₀-Methylester oder eine Mischung davon ist, das Lösungsmittel Methanol und der Katalysator Natriummethoxid ist.

Das Dokument WO 92/06158 betrifft eine gering schäumende Waschmittelzusammensetzung, umfassend mindestens 1 Gew.-% eines Polyhydroxyfettsäureamid-Tensids der Formel 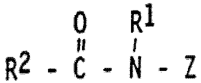 worin R¹ H, C₁-C₄-Kohlenwasserstoff, 2-Hydroxyethyl oder 2-Hydroxypropyl ist, R² Cs-C₃₁-Kohlenwasserstoff ist und Z ein Polyhydroxykohlenwasserstoff mit einer linearen Kohlenwasserstoffkette mit mindestens drei direkt mit der Kette verbundenen Hydroxylgruppen oder alkoxylierte Derivate davon ist; mindestens 1% eines alkylalkoxylierten Sulfat-Tensids; und wahlweise eine schaumunterdrückende Menge eines Schaumunterdrückers, welcher aus der monocarboxylische Fettsäuren und Salze davon, Silikon-Schaumunterdrücker und Monostearylkiakalimetallphosphaten oder-phosphatester und hochmolekulargewichtige Kohlenwasserstoff-Schaumunterdrücker und Mischungen hiervon umfassenden Gruppe gewählt ist; wobei die Zusammensetzung ein alkylalkoxyliertes Sulfat:Polyhydroxyfettsäureamid-Gewichtsverhältnis von 10:1 bis 1:10, vorzugsweise 5:1 bis 1:5 und stärker bevorzugt 4:1 bis 1:1 aufweist und das Polyhydroxyfettsäureamid weniger als 4 Gew.-% eines cyclischen Amid-Nebenprodukts umfasst.

WO 98/56496 betrifft eine Tensidzusammensetzung mit verbesserter Schaumstabilität. Die Tensidzusammensetzung enhält: (a) von ungefähr 1 bis ungefähr 40 Gew.-% eines Zuckertensids; (b) von ungefähr 1 bis ungefähr 40 Gew.-% eines anionischen Tensids; (c) von ungefähr 0,11 bis ungefähr 10 Gew.-% eines Amphoacetats; und (d) Rest Wasser, wobei sich die Gewichtsangaben auf das Gewicht der Zusammensetzung beziehen.

Die Aufgabe der Erfindung liegt somit darin, verbesserte Zusammensetzungen bereitzustellen, insbesondere im Hinblick auf ein verbessertes Schaumverhalten.

Überraschenderweise wurde gefunden, dass Formulierungen, enthaltend N-Acyl-N-Methylglucamine mit einer Kettenverteilung des natürlichen Kokos- oder Palmkernöls in bezug auf die Schaumeigenschaften den in der WO 92/06158 genannten C12/14 N-Acyl-N-Methylglucaminen überlegen sind. Besondere Vorteile weisen hierbei jene N-Acyl-N-methylglucamine auf, die aus Triglyceridöl hergestellt wurden und dementsprechend Glycerin und geringe Mengen Glycerinderivate enthalten.

Demgemäß wird eine Zusammensetzung bereitgestellt, enthaltend:
(A) mindestens ein anionisches Tensid als Komponente A,
(B) mindestens ein Betaintensid als Komponente B ausgewählt aus der Gruppe bestehend aus einem Alkylbetain, einem Alkylamidobetain und Mischungen daraus,
(C) eine Mischung aus N-Methyl-N-acylglucaminen als Komponente C, wobei die N-Methyl-N-acylglucamine einen C₈-C₂₂-Acylrest aufweisen und die Verteilung der C₈-C₂₂-Acylreste der des natürlichen Kokosöls oder Palmkernöls oder einer Mischung von beiden entspricht,
(D) mindestens ein Glycerin-Derivat als Komponte D, ausgewählt aus der Gruppe bestehend aus Glycerin, Monoglyceriden, Diglyceriden, Triglyceriden, wobei die jeweiligen Glyceride mindestens einen C₈-C₂₂-Acylrest aufweisen, und Mischungen daraus,
(E) mindestens ein Lösungsmittel als Komponente E und
(F) gegebenenfalls ein oder mehrere Additive als Komponente F;
dadurch gekennzeichnet, dass die Komponenten C und D durch die Umsetzung eines Triglycerids und N-Methylglucamin erhalten wurden.

Die erfindungsgemäße Zusammensetzung zeichnet sich insbesondere durch eine synergistische Wirkung der Komponenten C und D aus. Wie aus dem Vergleichsbeispiel ersichtlich ist, führt eine Zusammensetzung ohne die Komponente D nicht zu dem gleichen Effekt wie die erfindungsgemäße Zusammensetzung. Dies kann darauf zurückgeführt werden, dass die bei der Umsetzung von einem Triglycerid und einem N-Methylglucamin entstehenden Produkte, insbesondere in deren spezifischem Verhältnis, überraschenderweise zu den verbesserten Eigenschaften führen.

Das anionische Tensid der Komponente A kann beispielsweise ein Aminosäuretensid sein. Bevorzugt sind Acylglycinate, Acylalaninate, Acylaspartate, Acylglutamate und Acylsarkosinate, besonders Natrium Cocoyl-Glycinat..Kaliumcocoylglycinat, Natriumlauroylglycinat, Kaliumlauroylglycinat, Natriumcocoylglutamat, Natiiumlauroylglutamat, Natriumcocoylaspartat, Natriumlauroylaspartat und Natriumlauroylsarkosinat.

In einer bevorzugten Ausführungsform ist die Komponente A ausgewählt aus einer oder mehreren Verbindung(en) der allgemeinen Formel (I),

R¹SO₃⁻ M+ (I)

wobei R¹ für Alkyl, Cycloalkyl, Aralkyl, Aryl, Alkoxy, Alkoxyalkyl oder Heterocyclyl steht und M+ ein Alkalimetall-, Erdalkalimetall- oder ein substituiertes oder nicht substituiertes Ammoniumion ist,
und/oder der allgemeinen Formel (II),

R¹SO₄⁻ M+ (II)

wobei R¹ für Alkyl, Cycloalkyl, Aralkyl, Aryl, Alkoxy, Alkoxyalkyl und Heterocyclyl steht und M+ ein Alkalimetall-, Erdalkalimetall- oder ein substituiertes oder nicht substituiertes Ammoniumion ist.

"Alkyl" bedeutet eine gesättigte aliphatische Kohlenwasserstoffgruppe, die geradkettig oder verzweigt sein kann und von 1 bis 20 Kohlenstoffatome in der Kette haben kann. Bevorzugte Alkylgruppen können geradkettig oder verzweigt sein und von 1 bis zu 10 Kohlenstoffatomen in der Kette aufweisen. Verzweigt bedeutet, dass eine NiederAlkylgruppe, wie Methyl, Ethyl oder Propyl, an eine lineare Alkylkette angebracht ist. Bei Alkyl handelt es sich beispielsweise um Methyl, Ethyl, 1-Propyl, 2-Propyl, 1-Butyl, 2-Butyl, 2-Methyl-1-propyl (Isobutyl), 2-Methyl-2-propyl (tert.-Butyl), 1-Pentyl, 2-Pentyl, 3-Pentyl, 2-Methyl-1-butyl, 3-Methyl-1-butyl, 2-Methyl-2-butyl, 3-Methyl-2-butyl, 2,2-Dimethyl-1-propyl, 1-Hexyl, 2-Hexyl, 3-Hexyl, 2-Methyl-1-pentyl, 3-Methyl-1-pentyl, 4-Methyl-1-pentyl, 2-Methyl-2-pentyl, 3-Methyl-2-pentyl, 4-Methyl-2-pentyl, 2-Methyl-3-pentyl, 3-Methyl-3-pentyl, 2,2-Dimethyl-1-butyl, 2,3-Dimethyl-1-butyl, 3,3-Dimethyl-1-butyl, 2-Ethyl-1-butyl, 2,3-Dimethyl-2-butyl, 3,3-Dimethyl-2-butyl, 1-Heptyl, 1-Octyl, 1-Nonyl, 1-Decyl, 1-Undecyl, 1-Dodecyl, 1-Tetradecyl, 1-Hexadecyl und 1-Octadecyl. "Cycloalkyl" bedeutet einen aliphatischen Ring, der von 3 bis 10 Kohlenstoffatome in dem Ring hat. Bevorzugte Cycloalkylgruppen haben von 4 bis 7 Kohlenstoffatome in dem Ring.

"Aryl" bedeutet Phenyl oder Naphthyl.

"Aralkyl" bedeutet eine Alkylgruppe, die mit einem Arylrest substituiert ist.

"Substituiertes Aralkyl" und "substituiertes Aryl" bedeuten, dass die Arylgruppe oder die Alkylgruppe der Aralkylgruppe mit einem oder mehreren Substituenten ausgewählt aus Alkyl, Alkoxy, Nitro, Carboalkoxy, Cyano, Halo, Alkylmercaptyl, Trihaloalkyl oder Carboxyalkyl substituiert ist.

"Alkoxy" bedeutet eine Alkyl-O-Gruppe, in der "Alkyl" die vorstehend beschriebene Bedeutung hat. Nieder-Alkoxy-Gruppen sind bevorzugt. Beispiele sind Methoxy, Ethoxy, n-Propoxy, i-Propoxy und n-Butoxy.

"Nieder-Alkyl" bedeutet eine Alkylgruppe, die 1 bis 7 Kohlenstoffatome aufweist. "Alkoxyalkyl" bedeutet eine Alkylgruppe wie vorstehend beschrieben, die mit einer Alkoxygruppe, wie vorstehend beschrieben, substituiert ist. Somit kann unter dem Begriff Alkoxyalkyl ein Polyether verstanden werden.

"Heterocyclyl" bedeutet eine 4 bis 10-gliedrige Ringstruktur, in der ein oder mehrere Ringatome von Kohlenstoff verschieden sind, beispielsweise N, O oder S sind. Heterocyclyl kann aromatisch oder nicht-aromatisch sein, d. h. es kann gesättigt, teilweise oder ganz ungesättigt sein.

Besonders bevorzugt als Komponente A sind Natriumlaurylsulfat und/oder Natriumlaurylethersulfat.

Die Komponente B ist ausgewählt aus der Gruppe bestehend aus mindestens einem Alkylbetain, einem Alkylamidobetain oder Mischungen daraus.

Beispiele für geeignete Alkylbetaine stellen die Carboxyalkylierungsprodukte von sekundären und insbesondere tertiären Aminen der Formel (III) dar, 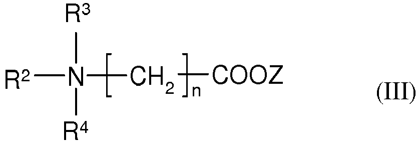 in der R² für Alkyl- und/oder Alkenylreste mit 6 bis 22 Kohlenstoffatomen, R³ für Wasserstoff oder Alkylreste mit 1 bis 4 Kohlenstoffatomen, R⁴ für Wasserstoff oder Alkylreste mit 1 bis 4 Kohlenstoffatomen, n für Zahlen von 1 bis 6 und Z für ein Alkali- und/oder Erdalkalimetall oder Ammonium steht. Typische Beispiele sind die Carboxymethylierungsprodukte von Hexylmethylamin, Hexyldimethylamin, Octyldimethylamin, Decyldimethylamin, Dodecylmethylamin, Dodecyldimethylamin, Dodecylethylmethylamin, C12/14-Kokosalkyldimethylamin, Myristyldimethylamin, Cetyldimethylamin, Stearyldimethylamin, Stearylethylmethylamin, Oleyldimethylamin, C16/18-Talgalkyldimethylamin sowie deren technische Gemische.

Beispiele für geeignete Alkylamidobetaine stellen Carboxyalkylierungsprodukte von Amidoaminen da. Insbesondere geeignet sind Amidopropylbetaine der Formel (IV), 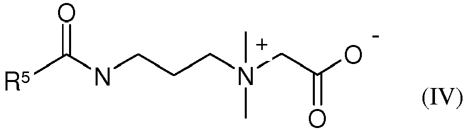 worin R⁵ eine lineare oder verzweigte gesättigte C₇-C₂₁ Alkylgruppe oder eine lineare oder verzweigte ein- oder mehrfach ungesättigte C₇-C₂₁ Alkenylgruppe ist.

Bevorzugte Betaintenside sind Amidopropylbetaine wie Cocoamidopropylbetain (R⁵CO ist der Fettsäurerest des Kokosöls, Kettenlänge C₈-C₁₈) und Alkylbetaine wie Koko-Betain (R² ist der Alkylrest des Kokosöls, Kettenlänge C₈-C₁₈) oder Laurylbetain (R² ist ein Alkylrest der Kettenlänge C₁₂ und C₁₄).

Beispielsweise und bevorzugt kann die N-Methyl-N-acylglucamin-Mischung (C) nach EP 0 550 637 aus den korrespondierenden Triglyceriden und N-Methylglucamin in Gegenwart von 1,2 Propylenglykol als Lösemittel hergestellt werden. Dabei werden in einer bevorzugten Ausführungsform durch Umamidierung der Triglyceride Kokosöl und/oder Palmkernöl mit N-Methylglucamin die Komponenten (C) und (D) gebildet.

Weitere Begriffe für N-Methyl-N-acylglucamin sind N-Methyl-N-1-Desoxysorbitol-Fettsäureamid, N-Acyl-N-methyl-glucamin, Glucamid oder N-Methyl-N-alkylglucamid. Dabei entspricht N-Methyl-N-acylglucamin der Formel (V), wobei R ein organischer Rest ist: 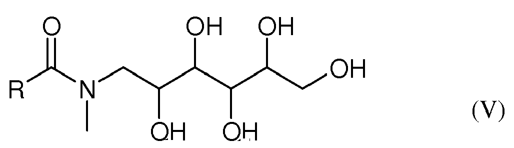 R-CO entspricht erfindungsgemäß den C₈-C₂₂-Acylresten des natürlichen Kokos- und/oder Palmkernöls.

In einer bevorzugten Ausführungsform entspricht die Verteilung der C₈-C₂₂-Acylreste in der N-Methyl-N-acylglucamin Mischung der von natürlichem Kokosöl.

Die Verteilung der C₈-C₂₂-Acylreste, die dem natürlichen Kokosöl entspricht, korrespondiert zu den C₈-C₂₂-Fettsäuren im Kokosöl. Dabei enthält Kokosöl Triglyceride.

Kokosöl umfasst dabei
a) 40-55 Gew.-% Laurinsäure,
b) 10-20 Gew.-% Myristinsäure,
c) 8-12 Gew.-% Palmitinsäure,
d) 6-12 Gew.-% Ölsäure und
h) 0-36 Gew.-% weiterer Fettsäuren,
wobei die Summe der an das Triglycerid gebundenen Fettsäuren 100 Gew.-% ergibt.

Besonders bevorzugt umfasst Kokosöl
a) 40-55 Gew.-% Laurinsäure,
b) 10-20 Gew.-% Myristinsäure,
c) 8-12 Gew.-% Palmitinsäure,
d) 6-12 Gew.-% Ölsäure,
e) 5-10 Gew.-% Decansäure,
f) 4-10 Gew.-% Octansäure,
g) 1-3 Gew.-% Stearinsäure und
h) 0-26 Gew.-% weiterer Fettsäuren,
wobei die Summe der an das Triglycerid gebundenen Fettsäuren 100 Gew.-% ergibt.

In einer weiteren bevorzugten Ausführungsform entspricht die Verteilung der C₈-C₂₂-Acylreste in der N-Methyl-N-acylglucamin Mischung der von natürlichem Palmkernöl. Die Verteilung der C₈-C₂₂-Acylreste, die dem natürlichen Palmkernöl entspricht, korrespondiert zu den C₈-C₂₂-Fettsäuren im Palmkernöl. Dabei enthält Palmkernöl Triglyceride.

Palmkernöl umfasst dabei
a) 45-55 Gew.-% Laurinsäure,
b) 14-18 Gew.-% Myristinsäure,
c) 6-10 Gew.-% Palmitinsäure,
d) 10-17 Gew.-% Ölsäure und
h) 0-25 Gew.-% weiterer Fettsäuren,
wobei die Summe der an das Triglycerid gebundenen Fettsäuren 100 Gew.-% ergibt.

Besonders bevorzugt umfasst Palmkernöl
a) 46-49 Gew.-% Laurinsäure,
b) 15-17 Gew.-% Myristinsäure,
c) 7-9 Gew.-% Palmitinsäure,
d) 13-15 Gew.-% Ölsäure,
e) 1-3 Gew.-% Decansäure,
f) 1-3 Gew.-% Octansäure,
g) 2-4 Gew.-% Stearinsäure und
h) 0-15 Gew.-% weiterer Fettsäuren,
wobei die Summe der an das Triglycerid gebundenen Fettsäuren 100 Gew.-% ergibt.

In einer weiteren bevorzugten Ausführungsform entspricht die Verteilung der C₈-C₂₂-Acylreste in der N-Methyl-N-acylglucamin-Mischung der einer Mischung von natürlichem Kokosöl und Palmkernöl.

Als Glycerin-Derivate D werden bevorzugt die bei der Umamidierung von Kokos- und/oder Palmkernöl mit N-Methylglycamin entstehenden eingesetzt. Als weitere Glycerin-Derivate kommen beispielweise Triglyceride von linearen oder verzweigten, gesättigten oder ungesättigten, gegebenenfalls hydroxylierten C8-C30-Fettsäuren in Betracht, insbesondere pflanzliche Öle, wie Sonnenblumen-, Mais-, Soja-, Reis-, Jojoba-, Babusscu-, Kürbis-, Traubenkern-, Sesam-, Walnuss-, Aprikosen-, Orangen-, Weizenkeim-,Pfirsichkern-, Makadamia-, Avocado-, Süßmandel, Wiesenschaumkraut-, Ricinus-, Oliven-, Erdnuss-, Raps- und Kokosnussöl, sowie synthetische Triglyceridöle. Auch gehärtete Triglyceride sind erfindungsgemäß bevorzugt. Auch Öle tierischen Ursprungs, beispielweise Rindertalg, Perhydrosqualen und Lanolin können eingesetzt werden. Neben den Triglyceriden können auch Mono- und Diglyceride eingesetzt werden.

Unter einem Lösungsmittel E im Rahmen der vorliegenden Erfindung versteht man vorzugsweise protische Lösungsmittel wie Wasser, C₁-C₈-Alkohole, insbesondere C₁-C₆-Alkohole, Ethylenglykol, Diethylenglykol, Triethylenglykol oder Mischungen davon, wobei insbesondere Wasser und/oder Ethanol oder Wasser und/oder Methanol bevorzugt sind. Von den C₁-C₆-Alkoholen sind Methanol, Ethanol, Isopropanol, n-Butanol oder sek.-Butanol bevorzugt.

Bevorzugtes Lösungsmittel ist Wasser oder Mischungen aus Wasser und Propylenglykol.

Im Rahmen einer bevorzugten Ausführungsform sind die Additive F - soweit vorhanden - gewählt aus der Gruppe bestehend aus Konservierungsmitteln, Duftstoffen, Farbstoffen, Tensiden, Wasser, Ölkörpern, kationische Polymeren, Filmbildnern, Verdickungs- und Gelierungsmitteln, Überfettungsmitteln, antimikrobiellen und biogenen Wirkstoffen, feuchtigkeitsspendenen Mitteln, Stabilisatoren, Säuren, Laugen, Wirkverstärkern und Mischungen daraus, bevorzugt in Mengen von 0,1 bis 10,0 Gew.-%, besonders bevorzugt von 0,5 bis 8,0 Gew.-% und insbesondere von 1,0 bis 5,0 Gew.-%.

Als Konservierungsmittel eignen sich die im betreffenden Annex der europäischen Kosmetikgesetzgebung gelisteten Konservierungsmittel, beispielsweise Phenoxyethanol, Benzylalkohol, Parabene, Benzoesäure und Sorbinsäure, besonders gut geeignet sind beispielsweise 1,3-Bis(hydroxymethyl)-5,5-dimethylimidazolidine-2,4-dione (Nipaguard^{®} DMDMH), Pirocton Olamin, Methylisothiazolinon oder Mischungen daraus, bevorzugt Pirocton, Olamin und/oder Methylisothiazolinon.

Als Duft- bzw. Parfümstoffe oder Öle können einzelne Riechstoffverbindungen, z. B. die synthetischen Produkte vom Typ der Ester, Ether, Aldehyde, Ketone, Alkohole und Kohlenwasserstoffe, verwendet werden. Riechstoffverbindungen vom Typ der Ester sind z. B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z. B. die linearen Alkanale mit 8 bis 18 C-Atomen, Citral, Citronellal, Citronellyloxyacetaldehyd, Cycllamenaldehyd, Hydroxycitronellal, Lilial und Bourgeonal, zu den Ketonen z. B. die Ionone, alpha-Isomethylionon und Methyl-cedrylketon, zu den Alkoholen Anethol, Citronellol, Eugenol, Geranion, Linalol, Phenylethylalkohol und Terpineol, zu den Kohlenwasserstoffen gehören hauptsächlich die Terpene und Balsame. Bevorzugt werden Mischungen verschiedener Riechstoffe verwendet, die gemeinsam eine ansprechende Duftnote erzeugen.
Parfümöle können auch natürliche Riechstoffgemische enthalten, wie sie aus pflanzlichen oder tierischen Quellen zugänglich sind, z. B. Pinien-, Citrus-, Jasmin-, Lilien-, Rosen-, oder Ylang-Ylang-Öl. Auch ätherische Öle geringerer Flüchtigkeit, die meist als Aromakomponenten verwendet werden, eignen sich als Parfümöle, z. B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl und Ladanumöl.

Die gewünschte Viskosität der Zusammensetzungen kann durch Zugabe von Verdickern und Gelierungsmittel eingestellt (erhöht oder erniedrigt) werden. In Betracht kommen vorzugsweise Celluloseether und andere Cellulosederivate (z. B. Carboxymethylcellulose, Hydroxyethylcellulose), Gelatine, Stärke und Stärkederivate, Natriumalginate, Fettsäurepolyethylenglykolester, Agar-Agar, Traganth oder Dextrinderivate, insbesondere Dextrinester. Des Weiteren eignen sich Metallsalze von Fettsäuren, bevorzugt mit 12 bis 22 C-Atomen, beispielsweise Natriumstearat, Natriumpalmitat, Natriumlaurat, Natriumarachidate, Natriumbehenat, Kaliumstearat, Kaliumpalmitat, Natriummyristat, Aluminiummonostearat, Hydroxyfettsäuren, beispielsweise 12-Hydroxystearinsäure, 16-Hydroxyhexadecanoylsäure; Fettsäureamide; Fettsäurealkanolamide; Dibenzalsorbit und alkohollösliche Polyamide und Polyacrylamide oder Mischungen solcher. Weiterhin können vernetzte und unvernetzte Polyacrylate wie Carbomer, Natriumpolyacrylate oder sulfonsäurehaltige Polymere wie Ammoniumacryloyldimethyltaurat/Vinylpyrrolidon (VP)-Copolymer Verwendung finden.

An antimikrobiellen Wirkstoffen kommen beispielsweise Cetyltrimethylammoniumchlorid, Cetyl-pyridiniumchlorid, Benzethoniumchlorid, Diisobutylethoxyethyl-dimethylbenzylammoniumchlorid, Natrium N-Laurylsarcosinat, Natrium-N-Palmethylsarcosinat, Lauroylsarcosin, N-Myristoylglycin, Kalium-N-Laurylsarcosin, Trimethylammoniumchlorid, Natriumaluminiumchlorohydroxylactat, Triethylcitrat, Tricetylmethylammoniumchlorid, 2,4,4'-Trichloro-2'-hydroxydiphenylether (Triclosan), Phenoxyethanol, 1,5-Pentandiol, 1,6-Hexandiol, 3,4,4'-Trichlorocarbanilid (Triclocarban), Diaminoalkylamid, beispielsweise L-Lysinhexadecylamid, Citratschwermetallsalze, Salicylate, Piroctose, insbesondere Zinksalze, Pyrithione und deren Schwermetallsalze, insbesondere Zinkpyrithion, Zinkphenolsulfat, Farnesol, Ketoconazol, Oxiconazol, Bifonazole, Butoconazole, Cloconazole, Clotrimazole, Econazole, Enilconazole, Fenticonazole, Isoconazole, Miconazole, Sulconazole, Tioconazole, Fluconazole, Itraconazole, Terconazole, Naftifine und Terbinafine, Selendisulfid und Octopirox, Iodopropynylbutylcarbamat, Methylchloroisothiazolinon, Methylisothiazolinon, Methyldibromo Glutaronitril, AgCl, Chloroxylenol, Na-Salz von Diethylhexylsulfosuccinat, Natriumbenzoat, sowie Phenoxyethanol, Benzylalkohol, Phenoxyisopropanol, Parabene, bevorzugt Butyl-, Ethyl-, Methyl- und Propylparaben, sowie deren Na-Salze, Pentandiol, 1,2-Octandiol, 2-Bromo-2-Nitropropan-1,3-diol, Ethylhexylglycerin, Benzylalkohol, Sorbinsäure, Benzoesäure, Milchsäure, Imidazolidinylharnstoff, Diazolidinylharnstoff, Dimethyloldimethylhydantoin (DMDMH), Na-Salz von Hydroxymethylglycinat, Hydroxyethylglycin der Sorbinsäure und Kombinationen dieser Wirksubstanzen zum Einsatz.

Die erfindungsgemäßen Zusammensetzungen können des Weiteren biogene Wirkstoffe ausgewählt aus Pflanzenextrakten, wie beispielsweise Aloe Vera, sowie Lokalanästhetika, Antibiotika, Antiphlogistika, Antiallergika, Corticosteroide, Sebostatika, Bisabolol®, Allantoin®, Phytantriol®, Proteine, Vitamine ausgewählt aus Niacin, Biotin, Vitamin B2, Vitamin B3, Vitamin B6, Vitamin B3 Derivaten (Salzen, Säuren, Estern, Amiden, Alkoholen), Vitamin C und Vitamin C Derivaten (Salzen, Säuren, Estern, Amiden, Alkoholen), bevorzugt als Natriumsalz des Monophosphorsäureesters der Ascorbinsäure oder als Magnesiumsalz des Phosphorsäureesters der Ascorbinsäure, Tocopherol und Tocopherolacetat, sowie Vitamin E und/oder dessen Derivate enthalten.

Als Stabilisatoren können Metallsalze von Fettsäuren, wie z. B. Magnesium-, Aluminium- und/oder Zinkstearat, eingesetzt werden.

Als feuchtigkeitsspendende Substanz stehen beispielsweise Isopropylpalmitat, Glycerin und/oder Sorbitol zu Verfügung.

Als Überfettungsmittel können vorzugsweise Lanolin und Lecithin, nicht ethoxylierte und polyethoxylierte oder acylierte Lanolin- und Lecithinderivate, Polyolfettsäureester, Mono-, Di- und Triglyceride und/oder Fettsäurealkanolamide, wobei die letzteren gleichzeitig als Schaumstabilisatoren dienen, verwendet werden, die bevorzugt in Mengen von 0,01 bis 10,0 Gew.-%, besonders bevorzugt von 0,1 bis 5,0 Gew.-% und insbesondere bevorzugt von 0,5 bis 3,0 Gew.-% eingesetzt werden.

Als Säuren oder Laugen zur pH-Wert Einstellung werden vorzugsweise Mineralsäuren, insbesondere HCl, anorganische Basen, insbesondere NaOH oder KOH, oder organische Säuren, insbesondere Zitronensäure, verwendet.

Im Rahmen einer bevorzugten Ausführungsform enthält die Zusammensetzung
(A) 5 - 15 Gew.-% der Komponente A,
(B) 1 - 10 Gew.-% der Komponente B,
(C) 1 - 10 Gew.-%der Komponente C,
(D) 0,2 - 2,4 Gew.-% der Komponente D,
(E) 5 - 92,8 Gew.-% der Komponente E, wobei die Komponente E ein protisches Lösungsmittel ist,
(F) 0 - 10 Gew.-% der Komponente F,
wobei die Summe der Komponenten A bis F 100 Gew.-% ergibt.

Bevorzugt besteht die Zusammensetzung aus
(A) 5 - 15 Gew.-% der Komponente A,
(B) 1 - 10 Gew.-% der Komponente B,
(C) 1 - 10 Gew.-%der Komponente C,
(D) 0,2 - 2,4 Gew.-% der Komponente D,
(E) 5 - 92,8 Gew.-% der Komponente E, wobei die Komponente E ein protisches Lösungsmittel ist,
(F) 0 - 10 Gew.-% der Komponente F,
wobei die Summe der Komponenten A bis F 100 Gew.-% ergibt.

Im Rahmen einer weiteren bevorzugten Ausführungsform enthält die Zusammensetzung
(A) 5 - 10 Gew.-% der Komponente A,
(B) 1 - 7 Gew.-% der Komponente B,
(C) 1 - 7 Gew.-% der Komponente C,
(D) 0,2 - 2.4 Gew.-% der Komponente D,
(E) 5 - 92,8 Gew.-% der Komponente E, wobei die Komponente D ein protisches Lösungsmittel ist,
(F) 0 - 10 Gew.-% der Komponente F,
wobei die Summe der Komponenten A bis F 100 Gew.-% ergibt.

Im Rahmen einer besonders bevorzugten Ausführungsform enthält die Zusammensetzung
(A) 7 - 10 Gew.-% der Komponente A,
(B) 1 - 5 Gew.-% der Komponente B,
(C) 1 - 5 Gew.-%der Komponente C,
(D) 0,1 - 1,2 Gew.-%der Komponente D,
(E) 5 - 90,8 Gew.-% der Komponente E, wobei die Komponente D ein protisches Lösungsmittel ist,
(F) 0,1 - 5 % Gew.-% der Komponente F,
wobei die Summe der Komponenten A bis F 100 Gew.-% ergibt.

Im Rahmen einer bevorzugten Ausführungsform beträgt die Summe der Komponenten A, B und C von 7 bis 20 Gew.-%, bevorzugt von 10 bis 18 Gew.-% und insbesondere von 10 bis 15 Gew.-%.

Bevorzugt ist ein Vehältnis von Komponente A: Komponente B: Komponente C von 2:1:1 bis 4:1:1. Besonders bevorzugt ist ein Verhältnis von 3:1:1.

Im Rahmen einer bevorzugten Ausführungsform handelt es sich bei der Zusammensetzung um eine kosmetische, dermatologische oder pharmazeutische Zusammensetzung.

Ein weiterer Gegenstand der Erfindung ist die Verwendung der erfindungsgemäßen Zusammensetzung als Shampoo, Gesichtsreiniger, Flüssigreiniger oder Duschbad.

Ein weiterer Gegenstand der Erfindung ist die nicht-therapeutische Verwendung der erfindungsgemäßen Zusammensetzung zur Behandlung oder Pflege der Haut.

Ein weiterer Gegenstand der Erfindung ist die Verwendung der erfindungsgemäßen Zusammensetzung zur Behandlung oder Pflege der Haare.

Weiterhin Gegenstand der Erfindung sind nicht-therapeutische Verfahren zur Pflege oder Behandlung von Haut oder Haaren, wobei die Haut beziehungsweise die Haare in Kontakt mit einer erfindungsgemäßen Zusammensetzung gebracht werden.

Die Erfindung wird durch die nachstehenden Beispiele näher erläutert:
Herstellbeispiel H1 bis H3, Beispiel 1 sowie Vergleichsbeispiele 1 bis 4.

Die im folgenden beschriebenen N-Acyl-N-methyl-glucamine wurden nach EP 0 550 637 aus den korrespondierenden Fettsäuremethylestern oder Triglyceriden und N-Methylglucamin in Gegenwart von 1,2 Propylenglykol als Lösemittel hergestellt und als Feststoff bestehend aus Aktivsubstanz und 1,2 Propylenglykol erhalten. Das in den Herstellbeispielen H1 - H3 erhaltene Material enthält den angegebenen Gehalt an 1,2 Propylenglykol und wird in den in Tabelle 2 gezeigten Beispielen ohne weitere Aufreinigung direkt eingesetzt.

**Tabelle 1**

| Herstellbeispiel | Methylester | Triglycerid | Aktivsubstanz (%) | 1,2-Propylen-glykol (%) | Schmelzpunkt (°C) |
|---|---|---|---|---|---|
| H1 | C12/14 | - | 90 | 10 | 85 |
| H2 | C8/18(Kocoyl) | - | 90 | 10 | 75 |
| H3 | - | C8/18 Kokosöl | 90 | 10 | 50 |

C12/14 bedeutet, dass der Methylester aus einem Gemisch aus Laurinsäuremethylester ( C₁₂-Acylrest) und Myristinsäuremethylester (C₁₄-Acylrest) besteht (Verhältnis 75 : 25). C8/C18 bedeutet, dass der Methylester aus einem der natürlichen Verteilung des Fettsäuren im Kokosöl (Octansäuremethylester, Decansäuremethylester, Laurinsäuremethylester und Myristinsäuremethylester, Palmitinsäuremethylester, Stearinsäuremethylester, Ölsäuremethylester, Verhältnis etwa: 4 : 7 : 48 : 20: 10 : 3 : 8) besteht.

Es wurden Tensidlösungen bestehend aus Natrium Laurylethersulfat (Genapol LRO liq., Clariant), Cocamidopropylbetain (Genagen CAB 818, Clariant) und Zuckertensiden gemäß der folgenden Tabelle 2 hergestellt und durch Zugabe von Kochsalz auf eine einheitliche Viskosität von 5000 mPas angepasst. Der Gesamttensidgehalt betrug jeweils 12 %.
Die Produkte wurden in einem trainierten Sensorikpanel mit 10 Teilnehmern bezüglich der Parameter Schaumqualität, Schaumcremigkeit, Anschäumverhalten und Schaummenge bewertet.

Dabei entspricht: ++ = sehr gut, + = gut o = befriedigend, - = ausreichend -- = unbefriedigend, wobei die Parameter nach Mittelwertbildung qualitativ gegen den Standard (Vergleichsbeispiel 4) bewertet wurden.

**Tabelle 2**

| Beispiel | Zuckertensid | Verhältnis Natriumlaurylethersulfat/Cocamidopropylbetain/Zuckertensid | Schaumqualität | Schaumcremigkeit | Anschäum -verhalten | Schaum menge |
|---|---|---|---|---|---|---|
| Vergleichs beispiel 1 | Herstellbeispiel H1 | 6:2:2 | o | o | o | + |
| Vergleichs beispiel 2 | Herstellbeispiel H2 | 6:2:2 | + | + | + | + |
| Beispiel 1 | Herstell-beispiel H3 | 6:2:2 | ++ | ++ | ++ | ++ |
| Vergleichs beispiel 4 | - | 7:3 | o | o | o | o |

Wie aus den erhaltenen Ergebnissen klar wird, führt der Einsatz von N-Acyl-N-methylglucaminen auf Basis von C8/18 Triglyceriden gemäß Beispiel 1 (z.B. Basis Kokosöl) im Vergleich zu einem entsprechenden Lauroylderivat (Vergleichsbeispiel 1) und einem Kokosderivat auf Methylesterbasis zu einer verbesserten sensorischen Bewertung des Schaumverhaltens der Formulierung gegenüber dem Stand der Technik.

## Patentansprüche

1. Zusammensetzung enthaltend:
(A) mindestens ein anionisches Tensid als Komponente A,
(B) mindestens ein Betaintensid als Komponente B ausgewählt aus der Gruppe bestehend aus einem Alkylbetain, einem Alkylamidobetain und Mischungen daraus,
(C) eine Mischung aus N-Methyl-N-acylglucaminen als Komponente C, wobei die N-Methyl-N-acylglucamine einen C₈-C₂₂-Acylrest aufweisen und die Verteilung der C₈-C₂₂-Acylreste der des natürlichen Kokosöls, Palmkernöls oder einer Mischung von beiden entspricht,
(D) mindestens ein Glycerin-Derivat als Komponte D, ausgewählt aus der Gruppe bestehend aus Glycerin, Monoglyceriden, Diglyceriden, Triglyceriden, wobei die jeweiligen Glyceride mindestens einen C₈-C₂₂-Acylrest aufweisen, und Mischungen daraus,
(E) mindestens ein Lösungsmittel als Komponente E und
(F) gegebenenfalls ein oder mehrere Additive als Komponente F;
**dadurch gekennzeichnet, dass** die Komponenten C und D durch die Umsetzung eines Triglycerids und N-Methylglucamin erhalten wurden.

2. Zusammensetzung nach Anspruch 1, **dadurch gekennzeichnet, dass** die Verteilung der C₈-C₂₂-Acylreste der des natürlichen Kokosöls entspricht.

3. Zusammensetzung nach Anspruch 1 oder 2, enthaltend:
(A) 5-15 Gew.-% der Komponente A,
(B) 1-10 Gew.-% der Komponente B,
(C) 1-10 Gew.-%der Komponente C,
(D) 0,2-2,4 Gew.-% der Komponente D,
(E) 5-92,8 Gew.-% der Komponente E, wobei die Komponente E ein protisches Lösungsmittel ist, und
(F) 0- 10 Gew.-% der Komponente F,
wobei die Summe der Komponenten A bis F 100 Gew.-% ergibt.

4. Zusammensatzung nach einem der Ansprüche 1 bis 3, **dadurch gekennzeichnet, dass** die Komponente A ausgewählt ist aus einer oder mehreren Verbindung(en) der allgemeinen Formel (I),
R¹SO₃⁻ M⁺ (I)
wobei R¹ für Alkyl, Cycloalkyl, Aralkyl, Aryl, Alkoxy, Alkoxyalkyl und Heterocyclyl steht und M⁺ ein Alkalimetall-, Erdalkalimetall- oder ein substituiertes oder nicht substituiertes Ammoniumion ist,
und/oder der allgemeinen Formel (II),
R¹SO₄⁻ M⁺ (II)
wobei R¹ für Alkyl, Cycloalkyl, Aralkyl, Aryl, Alkoxy, Alkoxyalkyl und Heterocyclyl steht und M⁺ ein Alkalimetall-, Erdalkalimetall- oder ein substituiertes oder nicht substituiertes Ammoniumion ist.

5. Zusammensetzung nach einem der Ansprüche 1 bis 4, **dadurch gekennzeichnet, dass** die Komponete E Wasser oder ein Gemisch aus Wasser und Propylenglykol ist.

6. Zusammensetzung nach einem der Ansprüche 3 bis 5, **dadurch gekennzeichnet, dass** die Summe der Komponenten A, B und C von 7 bis 35 Gew.-% beträgt.

7. Zusammensetzung nach einem der Ansprüche 1 bis 6, **dadurch gekennzeichnet, dass** die Additive F aus der Gruppe bestehend aus Konservierungsmitteln, Duftstoffen, Farbstoffen, weiteren Tensiden, Wasser, Ölkörpern, kationische Polymeren, Filmbildnern, Verdickungs- und Gelierungsmitteln, Überfettungsmitteln, antimikrobiellen und biogenen Wirkstoffen, feuchtigkeitsspendenen Mitteln, Stabilisatoren, Säuren, Laugen, Wirkverstärkern und Mischungen daraus ausgewählt sind.

8. Zusammensetzung nach einem der Ansprüche 1 bis 7, **dadurch gekennzeichnet, dass** es sich bei der Zusammensetzung um eine kosmetische, dermatologische oder pharmazeutische Zusammensetzung handelt.

9. Verwendung der Zusammensetzung nach einem der Ansprüche 1 bis 8 als Shampoo, Gesichtsreiniger, Flüssigreiniger oder Duschbad.

10. Nicht-therapeutische Verwendung der Zusammensetzung nach einem der Ansprüche 1 bis 8 zur Behandlung oder Pflege der Haut.

11. Verwendung der Zusammensetzung nach einem der Ansprüche 1 bis 8 zur Behandlung oder Pflege der Haare.

12. Nicht-therapeutisches Verfahren zur Pflege oder Behandlung der Haut, wobei die Haut in Kontakt mit einer Zusammensetzung nach einem der Ansprüche 1 bis 8 gebracht wird.

13. Verfahren zur Pflege oder Behandlung der Haare, wobei die Haare in Kontakt mit einer Zusammensetzung nach einem der Ansprüche 1 bis 8 gebracht werden.

14. Zusammensetzung nach einem der Ansprüche 1 bis 8 zur Verwendung als pharmazeutische Zusammensetzung.

15. Zusammensetzung nach Anspruch 14 zur Behandlung der Haut.

## Claims

1. Composition comprising:
(A) at least one anionic surfactant as component A,
(B) at least one betaine surfactant as component B selected from the group consisting of an alkyl betaine, an alkylamido betaine and mixtures thereof,
(C) a mixture of N-methyl-N-acylglucamines as component C, wherein the N-methyl-N-acylgluamines have a C₈-C₂₂-acyl radical and the distribution of the C₈-C₂₂-acyl radicals corresponds to that of natural coconut oil, palm kernel oil or a mixture of the two,
(D) at least one glycerol derivative as component D, selected from the group consisting of glycerol, monoglycerides, diglycerides, triglycerides, wherein the glycerides in question have at least one C₈-C₂₂-acyl radical, and mixtures thereof,
(E) at least one solvent as component E, and
(F) optionally one or more additives as component F;
**characterized in that** components C and D have been obtained by the reaction of a triglyceride and N-methylglucamine.

2. Composition according to Claim 1, **characterized in that** the distribution of the C₈-C₂₂-acyl radicals corresponds to that of natural coconut oil.

3. Composition according to Claim 1 or 2, comprising:
(A) from 5 to 15% by weight of component A,
(B) from 1 to 10% by weight of component B,
(C) from 1 to 10% by weight of component C,
(D) from 0.2 to 2.4% by weight of component D,
(E) from 5 to 92.8% by weight of component E, wherein component E is a protic solvent, and
(F) from 0 to 10% by weight of component F, wherein the sum of components A to F is 100% by weight.

4. Composition according to one of Claims 1 to 3, **characterized in that** component A is selected from one or more compound(s) of formula (I)
R¹SO₃⁻ M⁺ (I)
wherein R¹ represents alkyl, cycloalkyl, aralkyl, aryl, alkoxy, alkoxyalkyl and heterocyclyl and M⁺ is an alkali metal, alkaline earth metal or substituted or unsubstituted ammonium ion,
and/or of formula (II)
R¹SO₄⁻ M⁺ (II)
wherein R¹ represents alkyl, cycloalkyl, aralkyl, aryl, alkoxy, alkoxyalkyl and heterocyclyl and M⁺ is an alkali metal, alkaline earth metal or substituted or unsubstituted ammonium ion.

5. Composition according to one of Claims 1 to 4, **characterized in that** component E is water or a mixture of water and propylene glycol.

6. Composition according to one of Claims 3 to 5, **characterized in that** the sum of components A, B and C is from 7 to 35% by weight.

7. Composition according to one of Claims 1 to 6, **characterized in that** the additives F are selected from the group consisting of preservatives, fragrances, dyes, further surfactants, water, oily substances, cationic polymers, film-forming agents, thickeners and gelling agents, superfatting agents, antimicrobial and biogenic active ingredients, moisture-donating agents, stabilizers, acids, lyes, activity enhancers, and mixtures thereof.

8. Composition according to one of Claims 1 to 7, **characterized in that** the composition is a cosmetic, dermatological or pharmaceutical composition.

9. Use of the composition according to one of Claims 1 to 8 as a shampoo, facial cleanser, liquid cleanser or shower gel.

10. Non-therapeutic use of the composition according to one of Claims 1 to 8 for the treatment or care of the skin.

11. Use of the composition according to one of Claims 1 to 8 for the treatment or care of the hair.

12. Non-therapeutic method for caring for or treating the skin, wherein the skin is brought into contact with a composition according to one of Claims 1 to 8.

13. Method for caring for or treating the hair, wherein the hair is brought into contact with a composition according to one of Claims 1 to 8

14. Composition according to one of Claims 1 to 8 for use as a pharmaceutical composition.

15. Composition according to Claim 14 for the care of the skin.

## Revendications

1. Composition contenant :
(A) au moins un tensioactif anionique en tant que composant A,
(B) au moins un tensioactif de type bétaïne en tant que composant B, choisi dans le groupe constitué par une alkylbétaïne, une alkylamidobétaïne et des mélanges de ceux-ci,
(C) un mélange de N-méthyl-N-acylglucamines en tant que composant C, les N-méthyl-N-acylglucamines comportant un radical acyle en C₈-C₂₂ et la distribution des radicaux acyle en C₈-C₂₂ correspondant à celle de l'huile de coprah naturelle, de l'huile de palmiste naturelle ou d'un mélange des deux,
(D) au moins un dérivé de glycérol en tant que composant D, choisi dans le groupe constitué par le glycérol, les monoglycérides, diglycérides, triglycérides, les glycérides respectifs comportant au moins un radical acyle en C₈-C₂₂, et des mélanges de ceux-ci,
(E) au moins un solvant en tant que composant E et
(F) éventuellement un ou plusieurs additifs en tant que composant F ;
**caractérisée en ce que** les composants C et D ont été obtenus par la réaction d'un triglycéride et de N-méthylglucamine.

2. Composition selon la revendication 1, **caractérisée en ce que** la distribution des radicaux acyle en C₈-C₂₂ correspond à celle de l'huile de coprah naturelle.

3. Composition selon la revendication 1 ou 2, contenant :
(A) 5-15 % en poids du composant A,
(B) 1-10 % en poids du composant B,
(C) 1-10 % en poids du composant C,
(D) 0,2-2,4 % en poids du composant D,
(E) 5-92,8 % en poids du composant E, le composant E étant un solvant protique, et
(F) 0-10 % en poids du composant F,
la somme des composants A à F étant égale à 100 % en poids.

4. Composition selon l'une quelconque des revendications 1 à 3, **caractérisée en ce que** le composant A est choisi parmi un ou plusieurs composé(s) de formule générale (I),
R¹SO₃⁻ M⁺ (I)
dans laquelle R¹ représente un groupe alkyle, cycloalkyle, aralkyle, aryle, alcoxy, alcoxyalkyle et hétérocyclyle et M⁺ est un ion de métal alcalin, de métal alcalino-terreux ou un ion ammonium substitué ou non substitué,
et/ou de formule générale (II),
R¹SO₄⁻ M⁺ (II)
dans laquelle R¹ représente un groupe alkyle, cycloalkyle, aralkyle, aryle, alcoxy, alcoxyalkyle et hétérocyclyle et M⁺ est un ion de métal alcalin, de métal alcalino-terreux ou un ion ammonium substitué ou non substitué.

5. Composition selon l'une quelconque des revendications 1 à 4, **caractérisée en ce que** le composant E est l'eau ou un mélange d'eau et de propylèneglycol.

6. Composition selon l'une quelconque des revendications 3 à 5, **caractérisée en ce que** la somme des composants A, B et C vaut de 7 à 35 % en poids.

7. Composition selon l'une quelconque des revendications 1 à 6, **caractérisée en ce que** les additifs F sont choisis dans le groupe constitué par des conservateurs, des parfums, des colorants, d'autres tensioactifs, l'eau, des corps huileux, des polymères cationiques, des agents filmogènes, des épaississants et gélifiants, des agents de regraissage, des substances actives antimicrobiennes et biogènes, des agents hydratants, des stabilisants, des acides, des solutions alcalines, des activateurs et des mélanges de ceux-ci.

8. Composition selon l'une quelconque des revendications 1 à 7, **caractérisée en ce que** pour ce qui est de la composition il s'agit d'une composition cosmétique, dermatologique ou pharmaceutique.

9. Utilisation de la composition selon l'une quelconque des revendications 1 à 8, en tant que shampooing, nettoyant pour le visage, produit de nettoyage liquide ou gel douche.

10. Utilisation non thérapeutique de la composition selon l'une quelconque des revendications 1 à 8, pour le traitement ou le soin de la peau.

11. Utilisation de la composition selon l'une quelconque des revendications 1 à 8, pour le traitement ou le soin de la peau

12. Procédé non thérapeutique pour le soin ou le traitement de la peau, dans lequel on met la peau en contact avec une composition selon l'une quelconque des revendications 1 à 8.

13. Procédé pour le soin ou le traitement des cheveux, dans lequel on met les cheveux en contact avec une composition selon l'une quelconque des revendications 1 à 8.

14. Composition selon l'une quelconque des revendications 1 à 8, pour utilisation en tant que composition pharmaceutique.

15. Composition selon la revendication 14, pour le traitement de la peau.
